(19) Europäisches Patentamt
European Patent Office
Office européen des brevets

(11) **EP 1 847 547 A1**

(12) **DEMANDE DE BREVET EUROPEEN**

(43) Date de publication:
**24.10.2007 Bulletin 2007/43**

(51) Int Cl.:
***C07H 19/16*** *(2006.01)* ***A61Q 19/08*** *(2006.01)*
***A61K 8/49*** *(2006.01)*

(21) Numéro de dépôt: **07105862.2**

(22) Date de dépôt: **10.04.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **11.04.2006 FR 0651327**

(71) Demandeur: **L'Oréal**
**75008 Paris (FR)**

(72) Inventeurs:
- **Dalko, Maria**
**91190, GIF S/YVETTE (FR)**
- **Fagot, Dominique**
**75020, PARIS (FR)**

(74) Mandataire: **Kromer, Christophe**
**L'OREAL - D.I.P.I.**
**25-29 Quai Aulagnier**
**92600 Asnières (FR)**

(54) **Composition cosmétique antirides**

(57) La présente invention concerne l'utilisation cosmétique d'au moins un dérivé d'adénosine de formule (I) :

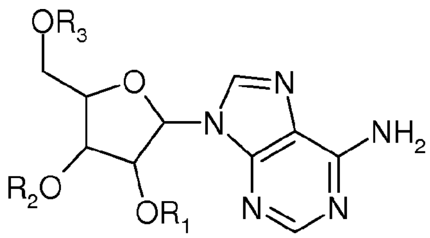

(I)

dans laquelle :
- (a) $R_1$ et $R_2$ identiques désignent un radical hydrocarboné en $C_1$-$C_6$ ou forment ensemble, avec les atomes d'oxygène auxquels ils sont rattachés un radical isopropylidène ;
- $R_3$ désigne :
(i) un groupe -$COR_4$ avec $R_4$ désignant un radical hydrocarboné en $C_1$-$C_9$, éventuellement substitué ;

ou (ii) un groupe ester issu de la biotine ;
ou
- (b) $R_1$ et $R_2$ forment ensemble, avec les atomes d'oxygène auxquels ils sont rattachés un radical isopropylidène ; et $R_3$ désigne un radical hydrocarboné en $C_1$-$C_{10}$ éventuellement substitué ;
R' et R" désignant un atome d'hydrogène, un radical hydrocarboné en $C_1$-$C_6$, éventuellement substitué
et leurs sels, isomères optiques et solvates,
comme agent pour lutter contre les rides, notamment les rides d'expression, et/ou décontracter la peau et/ou détendre les traits de la peau.

L'invention concerne également une composition cosmétique contenant un tel composé ainsi que les composés nouveaux correspondants.

EP 1 847 547 A1

## Description

La présente invention concerne un procédé de traitement cosmétique d'une peau ridée, comprenant l'application topique sur la peau d'une composition comprenant un dérivé d'adénosine. Elle concerne également de nouveaux dérivés d'adénosine.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation, des fibres élastiques qui composent le tissu cutané.

Bien que ces traitements permettent d'agir sur les rides et ridules dues au vieillissement chronologique ou intrinsèque, ainsi que sur celles dues au photo-vieillissement, ils n'ont pas d'effet sur les rides d'expression, lesquelles nécessitent une intervention sur la composante contractile musculaire (via des agents myorelaxants) ou dermique (via des agents dermo-décontractant) des rides.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet anti-rides lorsqu'ils sont appliqués topiquement sur la peau, permettant ainsi d'agir par une autre voie sur les rides d'expression.

Il reste toutefois le besoin de disposer d'autres composés efficaces pour lisser ou estomper les rides, en particulier d'expression.

Or, la Demanderesse a découvert avec étonnement que certains dérivés d'adénosine permettaient de satisfaire ce besoin.

La présente invention a donc pour objet l'utilisation cosmétique d'au moins un dérivé d'adénosine de formule (1) :

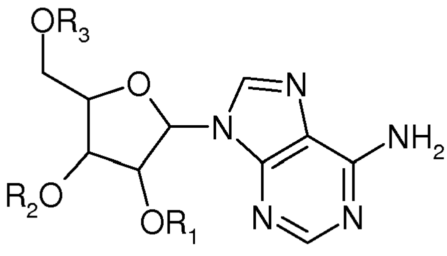

(I)

dans laquelle :

- (a) $R_1$ et $R_2$ identiques désignent un radical hydrocarboné linéaire saturé en $C_1$-$C_6$ ou insaturé en $C_2$-$C_6$, ou ramifié en $C_3$-$C_6$ saturé ou insaturé, ou forment ensemble, avec les atomes d'oxygène auxquels ils sont rattachés un radical isopropylidène;
  et $R_3$ désigne :

  (i) un groupe $-COR_4$ avec $R_4$ désignant un radical hydrocarboné linéaire saturé en $C_1$-$C_9$ ou insaturé en $C_2$-$C_9$,

ou ramifié en $C_3$-$C_9$ saturé ou insaturé, éventuellement substitué par au moins un groupement choisi parmi -OR', -NR'R", -COOR', -CONR'R", -CF3, -F , -$OCF_3$, -CN, -$NO_2$;

ou (ii) un groupe ester issu de la biotine;
ou

- (b) $R_1$ et $R_2$ forment ensemble, avec les atomes d'oxygène auxquels ils sont rattachés, un radical isopropylidène ; et $R_3$ désigne un radical hydrocarboné linéaire saturé en $C_1$-$C_{10}$ ou insaturé en $C_2$-$C_{10}$, ou ramifié en $C_3$-$C_{10}$ saturé ou insaturé, éventuellement substitué par au moins un groupement choisi parmi -OR', -NR'R", - COOR', -CONR'R", -$CF_3$, -F , -$OCF_3$, -CN, -$NO_2$;

R' et R" désignant un atome d'hydrogène, un radical hydrocarboné linéaire saturé en $C_1$-$C_6$ ou insaturé en $C_2$-$C_6$, ou ramifié en $C_3$-$C_6$ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en $C_1$-$C_6$ ou insaturé en $C_2$-$C_6$, ou ramifié en $C_3$-$C_6$ saturé ou insaturé ;
et leurs sels, isomères optiques et solvates,
comme agent pour lutter contre les rides, notamment les rides d'expression, et/ou décontracter la peau et/ou détendre les traits de la peau.

L'invention a encore pour objet un procédé de traitement cosmétique d'une peau ridée, en particulier la peau du visage et/ou du front, comprenant l'application topique sur ladite peau d'une composition comprenant, dans un milieu physiologiquement acceptable, au moins un dérivé d'adénosine de formule (I) tel que défini précédemment.

Le 2', 3'-isopropylidène- 5'-acétyl-adénosine est un composé connu, notamment décrit dans la demande WO-A-2004/037159 (composé 265 page 203) dans une composition pharmaceutique pour le traitement de l'obésité. Ce document ne décrit pas d'appliquer la composition par voie topique sur la peau, notamment pour traiter les rides.

L'invention a aussi pour objet une composition comprenant, dans un milieu physiologiquement acceptable, au moins un dérivé d'adénosine de formule (II):

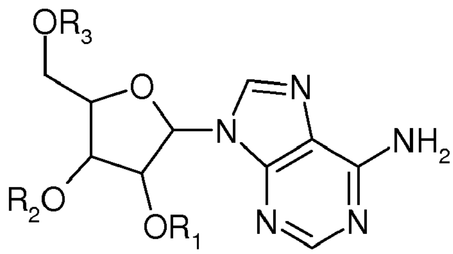

(II)

dans laquelle :

- (a) $R_1$ et $R_2$ identiques désignent un radical hydrocarboné linéaire saturé en $C_1$-$C_6$ ou insaturé en $C_2$-$C_6$ , ou ramifié en $C_3$-$C_6$ saturé ou insaturé, ou forment ensemble, avec les atomes d'oxygène auxquels ils sont rattachés un radical isopropylidène ;
  et $R_3$ désigne :

  (i) un groupe -$COR_4$ avec $R_4$ désignant un radical hydrocarboné linéaire saturé en $C_2$-$C_9$ ou insaturé en $C_2$-$C_9$ , ou ramifié en $C_3$-$C_9$ saturé ou insaturé, éventuellement substitué par au moins un groupement choisi parmi -OR', -NR'R", -COOR', -CONR'R", -CF3, -F , -$OCF_3$, -CN, -$NO_2$;

  ou (ii) un groupe ester issu de la biotine;

  ou

- (b) $R_1$ et $R_2$ forment ensemble, avec les atomes d'oxygène auxquels ils sont rattachés, un radical isopropylidène ; et $R_3$ désigne un radical hydrocarboné linéaire saturé en $C_1$-$C_{10}$ ou insaturé en $C_2$-$C_{10}$, ou ramifié en $C_3$-$C_{10}$ saturé ou insaturé, éventuellement substitué par au moins un groupement choisi parmi -OR', -NR'R", - COOR', -CONR'R",

$-CF_3$, -F , $-OCF_3$, -CN, $-NO_2$;

R' et R" désignant un atome d'hydrogène, un radical hydrocarboné linéaire saturé en $C_1$-$C_6$ ou insaturé en $C_2$-$C_6$, ou ramifié en $C_3$-$C_6$ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en $C_1$-$C_6$ ou insaturé en $C_2$-$C_6$ , ou ramifié en $C_3$-$C_6$ saturé ou insaturé ;
et leurs sels, isomères optiques et solvates.

Certains des composés de formule (I) sont connus de l'art antérieur et décrits dans les documents suivants:

- WO-A-2004/037159;
- Poppe, L et al ; "Synthesis and characterization of (5'-deoxyadenosin-5'-yl)cobalamin (='adenosylcobalamin') analogs mimicking the transition-state geometry of coenzyme-B12-dependent rearrangements" ; Helvetica Chimica Acta (1993), 76(6), 2367-83 ;
- Jones, A. S. et al; "Synthetic analogs of polynucleotides. VII. Syntheses of 5'-O-acryloylnucleosides and copolymers of these with other acryloyl compounds" ; Journal of the Chemical Society [Section] C: Organic (1971), (19), 3183-7 ;
- Mornet, D. et al ; "The reaction of myosin with a bromoalkyl analog of adenosine triphosphate" ; FEBS Letters (1977), 84(2), 362-6 ;
- Huber Gerhard ; "esters of adenosine with organic and inorganic" acids; Chem. Ber. 89, 2853-62 (1956) - ref CA52: 2027g
- Takemoto, K. et al; "Nucleic acid analogs: their specific interaction and applicability" ; Polymeric Materials Science and Engineering (1988), 58, 250-3 ;
- Purkayastha, Bhupesh C.; Bhattacharyya, S. N ; "Use of Ca oxalate monohydrate in the investigation of rare earth and thorium activities" ; J. Indian. Chem. Soc. 34, 427-33 (1957) - ref CA52:2627h ;
- Peterli, Stefan et al ; "Nitrostyrene derivatives of adenosine 5'-glutarates as selective inhibitors of the epidermal growth factor receptor protein tyrosine kinase" ; Helvetica Chimica Acta (1992). 75(3), 696-706.

L'invention a encore pour objet de nouveaux composés de formule (III):

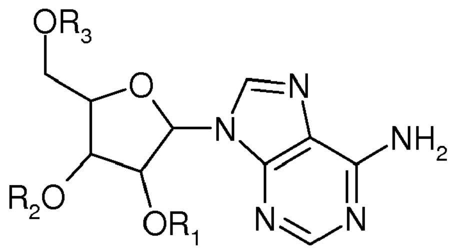

(III)

dans laquelle :

- (a) $R_1$ et $R_2$ identiques désignent un radical hydrocarboné linéaire saturé en $C_1$-$C_6$ ou insaturé en $C_2$-$C_6$, ou ramifié en $C_3$-$C_6$ saturé ou insaturé, ou bien encore forment ensemble, avec les atomes d'oxygène auxquels ils sont rattachés un radical isopropylidène ;
- $R_3$ désigne :

  (i) un groupe $-COR_4$ avec $R_4$ désignant un radical hydrocarboné linéaire en $C_3$-$C_9$ ou ramifié en $C_3$-$C_9$, saturé ou insaturé, éventuellement substitué par au moins un groupement choisi parmi -OR', -NR'R", -CONR'R", -CF3, -F , $-OCF_3$, -CN, $-NO_2$, $R_4$ ne désignant pas un groupe 2-amino éthyle;

ou (ii) un groupe ester issu de la biotine;

ou

- (b) $R_1$ et $R_2$ forment ensemble, avec les atomes d'oxygène auxquels ils sont rattachés, un radical isopropylidène ; et $R_3$ désigne un radical hydrocarboné linéaire en $C_2$-$C_{10}$ ou ramifié en $C_3$-$C_{10}$, saturé ou insaturé, éventuellement substitué par au moins un groupement choisi parmi -NR'R", -COOR', -CONR'R", $-CF_3$, -F , $-OCF_3$, - CN, $-NO_2$;

R' et R" désignant un atome d'hydrogène, un radical hydrocarboné linéaire saturé en $C_1$-$C_6$ ou insaturé en $C_2$-$C_6$ , ou un radical ramifié en $C_3$-$C_6$ saturé ou insaturé, éventuellement substitué par au moins un groupement choisi parmi -OZ, -NZZ', - COOZ, Z et Z' désignant, indépendamment l'un de l'autre, un atome d'hydrogène ou radical hydrocarboné linéaire saturé en $C_1$-$C_6$ ou insaturé en $C_2$-$C_6$ , ou ramifié en $C_3$-$C_6$ saturé ou insaturé,
et leurs sels, isomères optiques et solvates.

Dans la formule (I), les groupes hydrocarbonés (ou alkyle) peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle.

Des radicaux $R_1$ et $R_2$ préférés dans les formule (I) à (III) décrites précédemment sont ceux formant ensemble, avec les atomes d'oxygène auxquels ils sont rattachés, un radical isopropylidène.

Pour les composés de formule (I), on préfère ceux ayant les significations suivantes :

- $R_1$ et $R_2$ forment ensemble, avec les atomes d'oxygène auxquels ils sont rattachés, un radical isopropylidène ;
- $R_3$ désigne :

  (i) un radical hydrocarboné linéaire saturé en $C_1$-$C_{10}$ ou insaturé en $C_2$-$C_{10}$, ou ramifié en $C_3$-$C_{10}$, saturé ou insaturé ;

  ou (ii) un groupe $-COR_4$ avec $R_4$ désignant un radical hydrocarboné linéaire en $C_1$-$C_9$ ou ramifié en $C_3$-$C_9$, saturé ou insaturé ;

  ou (iii) un groupe ester issu de la biotine.

Préférentiellement, on utilise des composés de formule (I) pour lesquels:

- $R_1$ et $R_2$ forment ensemble, avec les atomes d'oxygène auxquels ils sont rattachés, un radical isopropylidène;
- $R_3$ désigne :

  (i) un radical hydrocarboné linéaire en $C_1$-$C_{10}$;
  ou (ii) un groupe $-COR_4$ avec $R_4$ désignant un radical hydrocarboné linéaire saturé en $C_1$-$C_9$;
  ou (iii) un groupe ester issu de la biotine.

Plus préférentiellement, on utilise des composés de formule (I) pour lesquels:

$R_1$ et $R_2$ forment ensemble, avec les atomes d'oxygène auxquels ils sont rattachés, un radical isopropylidène; $R_3$ désigne :

- un groupe $-COR_4$ avec $R_4$ désignant un radical hydrocarboné linéaire en $C_1$-$C_9$;
- ou un groupe ester issu de la biotine.

Pour les composés de formule (II), on préfère ceux ayant les significations suivantes:

$R_1$ et $R_2$ forment ensemble, avec les atomes d'oxygène auxquels ils sont rattachés, un radical isopropylidène;
$R_3$ désigne :

(i) un radical hydrocarboné linéaire saturé en $C_1$-$C_{10}$ ou insaturé en $C_2$-$C_{10}$, ou ramifié en $C_3$-$C_{10}$ saturé ou insaturé ;
ou (ii) un groupe $-COR_4$ avec $R_4$ désignant un radical hydrocarboné linéaire en $C_2$-$C_9$ ou ramifié en $C_3$-$C_9$, saturé ou insaturé;
ou (iii) un groupe ester issu de la biotine.

Préférentiellement, on utilise des composés de formule (II) pour lesquels:

$R_1$ et $R_2$ forment ensemble, avec les atomes d'oxygène auxquels ils sont rattachés, un radical isopropylidène; $R_3$ désigne :

(i) un radical hydrocarboné linéaire en $C_1$-$C_{10}$;

ou (ii) un groupe -$COR_4$ avec $R_4$ désignant un radical hydrocarboné linéaire saturé en $C_2$-$C_9$;

ou (iii) un groupe ester issu de la biotine.

Plus préférentiellement, on utilise des composés de formule (II) pour lesquels :

$R_1$ et $R_2$ forment ensemble, avec les atomes d'oxygène auxquels ils sont rattachés, un radical isopropylidène ; $R_3$ désigne :

- un groupe -$COR_4$ avec $R_4$ désignant un radical hydrocarboné linéaire en $C_2$-$C_9$ ;
- ou un groupe ester issu de la biotine.

Pour les composés de formule (III), on préfère ceux ayant les significations suivantes :

$R_1$ et $R_2$ forment ensemble, avec les atomes d'oxygène auxquels ils sont rattachés, un radical isopropylidène ; $R_3$ désigne :

(i) un radical hydrocarboné linéaire en $C_2$-$C_{10}$ ou ramifié en $C_3$-$C_{10}$, saturé ou insaturé ;
ou (ii) un groupe -$COR_4$ avec $R_4$ désignant un radical hydrocarboné linéaire en $C_3$-$C_9$ ou ramifié en $C_3$-$C_9$, saturé ou insaturé ;
ou (iii) un groupe ester issu de la biotine.

Préférentiellement, on utilise des composés de formule (III) pour lesquels :

$R_1$ et $R_2$ forment ensemble, avec les atomes d'oxygène auxquels ils sont rattachés, un radical isopropylidène ; $R_3$ désigne :

(i) un radical hydrocarboné linéaire en $C_2$-$C_{10}$ ;
ou (ii) un groupe -$COR_4$ avec $R_4$ désignant un radical hydrocarboné linéaire saturé en $C_3$-$C_9$ ;
ou (iii) un groupe ester issu de la biotine.

Plus préférentiellement, on utilise des composés de formule (III) pour lesquels :

$R_1$ et $R_2$ forment ensemble, avec les atomes d'oxygène auxquels ils sont rattachés, un radical isopropylidène ; $R_3$ désigne :

- un groupe -$COR_4$ avec $R_4$ désignant un radical hydrocarboné linéaire en $C_3$-$C_9$ ;
- ou un groupe ester issu de la biotine.

Comme sels des composé de formule (I) à (III), on peut citer les sels obtenus par addition de composé de formule (I), (II) ou (III) avec un acide inorganique, choisi notamment parmi les acides chlorhydrique, sulfurique et phosphorique, ou avec un acide organique, choisi en particulier parmi les acides acétique, propionique, succinique, fumarique, lactique, glycolique, citrique et tartrique.
De préférence, les sels des composés (I) , (II) ou (III) sont choisis parmi les sels obtenus à partir de l'acide chlorhydrique ou l'acide acétique ou l'acide citrique.
Les composés (I), (II) ou (III) peuvent être préparés selon l'un des quatre procédé de synthèse décrits ci-après en fonction de la signification des radicaux $R_1$ , $R_2$ et $R_3$.

Premier procédé

Les composés de formule (I) pour lesquels $R_1$ = $R_2$ et $R_3$ désignent un radical hydrocarboné tels que définis

précédemment, peuvent notamment être préparés selon le schéma I réactionnel suivant :

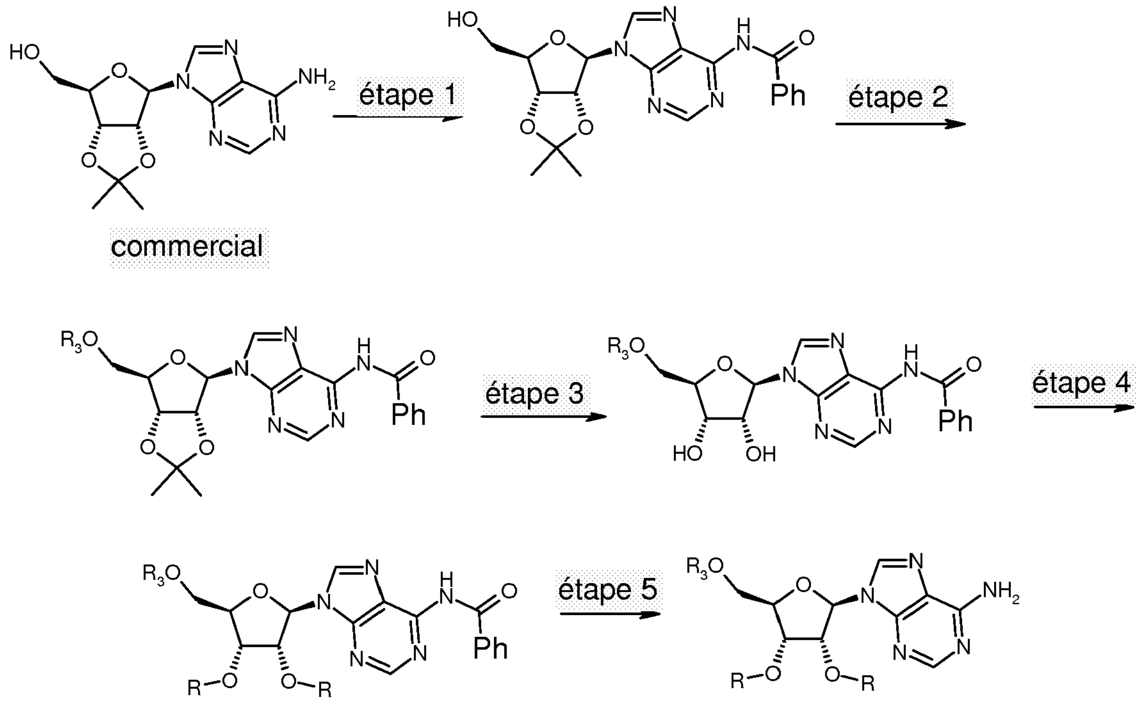


Schéma I

Une telle méthode de préparation est notamment décrite dans Helvetica Chimica Acta, 1993, (vol 76), page 2367.

Selon l'étape 1, on fait réagir l'isopropylidène adénosine avec le chlorotriméthylsilane, notamment dans la pyridine, puis on ajoute 1,2 équivalent molaire de chlorure de benzoyle. Après formation du dérivé N-benzoyle correspondant, on ajoute du fluorure de sodium dans un mélange eau/méthanol en milieu acide.

Selon l'étape 2, on ajoute au composé obtenu de l'hydrure de sodium dans le diméthylformamide et on ajoute un tosylate de formule $R_3OTs$ (Ts désigne le groupe tosyle) ou un composé halogéné de formule $R_3X$ (avec X désignant Cl, Br ou I).

Selon l'étape 3, on ajoute une solution aqueuse à 10 % d'acide chlorhydrique et du méthanol et on porte le mélange à reflux pendant 10 minutes.

Selon l'étape 4, on ajoute de l'hydrure de sodium dans le diméthylformamide puis 2 équivalents molaires de composé tosylate de formule $R_3OTs$ (Ts désigne le groupe tosyle) ou de composé halogéné de formule $R_3X$ (avec X désignant Cl, Br ou I).

Selon l'étape 5, on ajoute une quantité catalytique de méthylate de sodium dans du méthanol.

<u>Deuxième procédé</u>

Les composés de formule (I) pour lesquels $R_1$ et $R_2$ forment ensemble un radical ispropylidène et $R_3$ désigne un radical hydrocarboné tel que défini précédemment, peuvent notamment être préparés selon le schéma II réactionnel suivant :

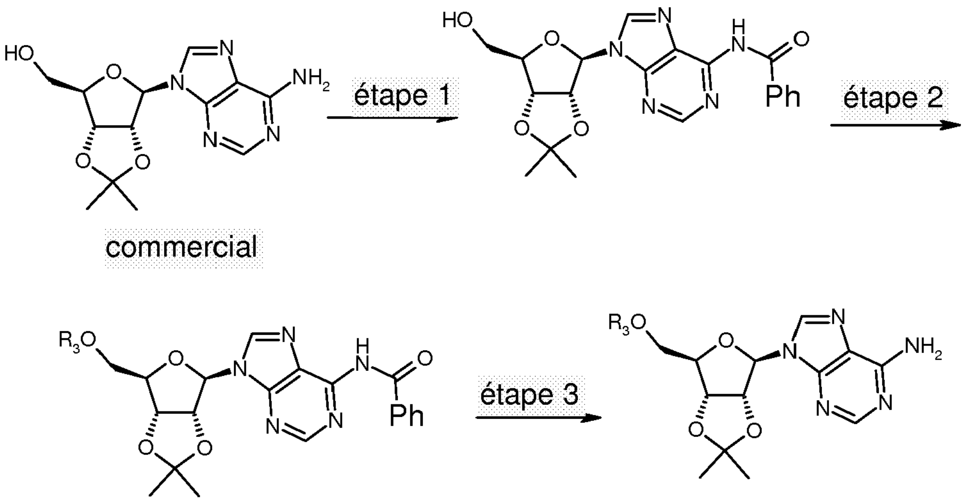


Schéma II

Selon l'étape 1, on fait réagir l'isopropylidène adénosine avec le chlorotriméthylsilane, notamment dans la pyridine, puis on ajoute 1,2 équivalent molaire de chlorure de benzoyle. Après formation du dérivé N-benzoyle correspondant, on ajoute du fluorure de sodium dans un mélange eau/méthanol en milieu acide.

Selon l'étape 2, on ajoute au composé obtenu de l'hydrure de sodium dans le diméthylformamide et on ajoute un tosylate de formule $R_3OTs$ (Ts désigne le groupe tosyle) ou un composé halogéné de formule $R_3X$ (avec X désignant Cl, Br ou I).

Selon l'étape 3, on ajoute une quantité catalytique de méthylate de sodium dans du méthanol.

Troisième procédé

Les composés de formule (I) pour lesquels $R_1 = R_2$ et désignent un radical hydrocarboné et $R_3$ désignant un radical $-COR_4$ ou un groupe ester issu de la biotine, tels que définis précédemment, peuvent notamment être préparés selon le schéma III réactionnel suivant :

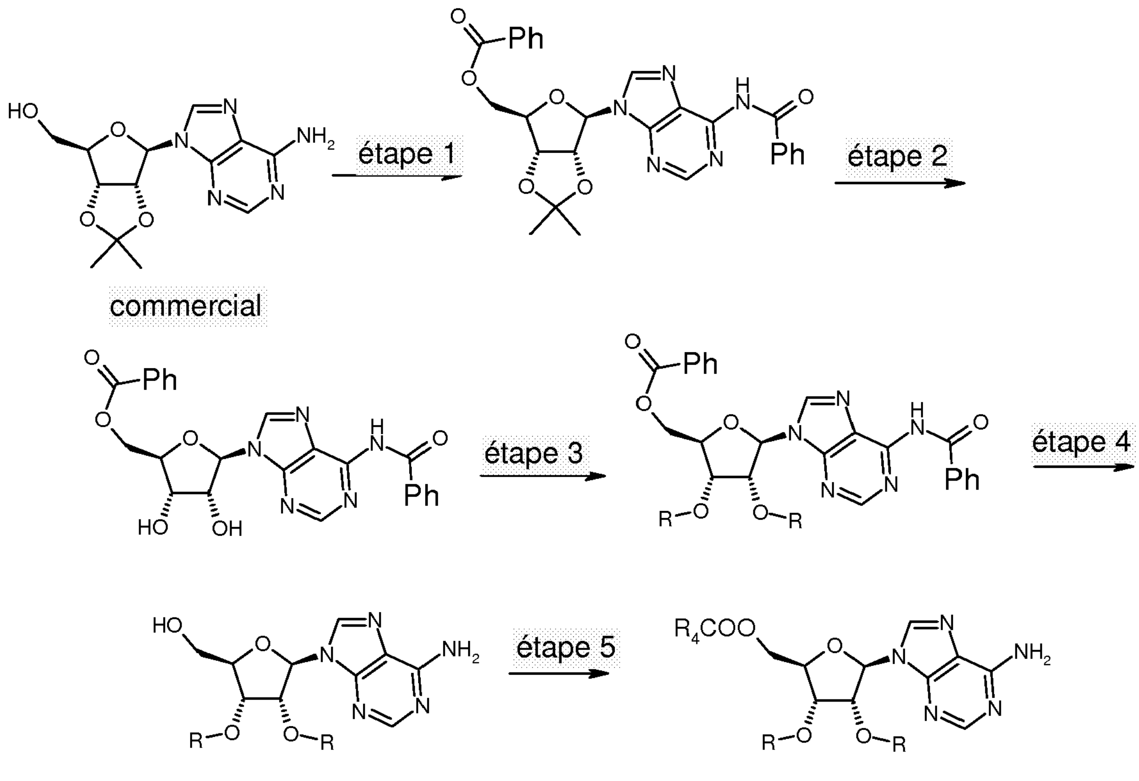


Schéma III

Selon l'étape 1, on fait réagir l'isopropylidène adénosine avec 2,1 équivalents molaires de chlorure de benzoyle , notamment dans la pyridine.

Puis selon l'étape 2, on ajoute une solution aqueuse à 10 % d'acide chlorhydrique et du méthanol et on porte le mélange à reflux pendant 10 minutes.

Selon l'étape 3, on ajoute de l'hydrure de sodium dans le diméthylformamide puis 2 équivalents molaires de composé halogéné de formule $R_3X$ (avec X désignant Cl, Br ou I).

Selon l'étape 4, on ajoute une quantité catalytique de méthylate de sodium dans du méthanol.

Selon l'étape 5, on ajoute un acide organique de formule $R_4COOH$ en présence de carbodiimidazole dans le diméthylformamide à une température d'environ 40 °C.

<u>Quatrième procédé</u>

Les composés de formule (I) pour lesquels $R_1$ et $R_2$ forment ensemble un radical ispropylidène et $R_3$ désignant un radical $-COR_4$ ou un groupe ester issu de la biotine, tels que définis précédemment, peuvent notamment être préparés selon le schéma IV réactionnel suivant :

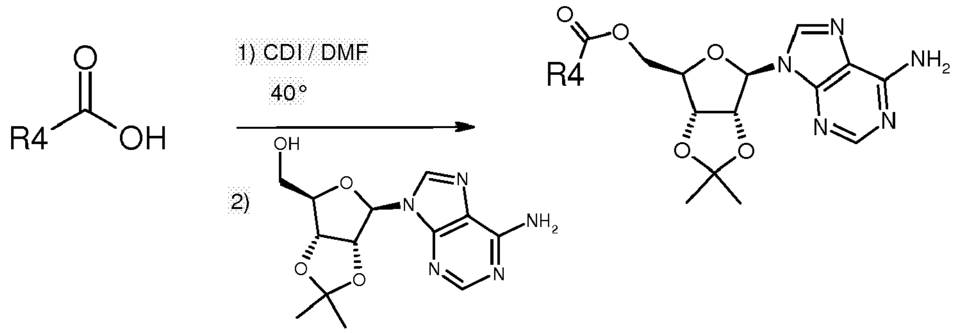


Schéma IV

On fait réagir l'acide carboxylique $R_4COOH$ en présence de carboxidiimide (CDI) dans le diméthylformamide à une température d'environ 40 °C et on ajoute l'isopropylidène adénosine.

Comme composés nouveaux de formule (III) (qui font également partie des composés de formule (I) et (II) décrits précédemment), on peut citer les composés suivants :

Composé A : 2',3'-isopropylidène-5'-butanoyle-adénosine

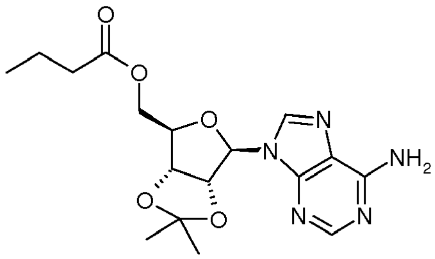

Composé B : 2',3'-isopropylidène-5'-octanoyle-adénosine

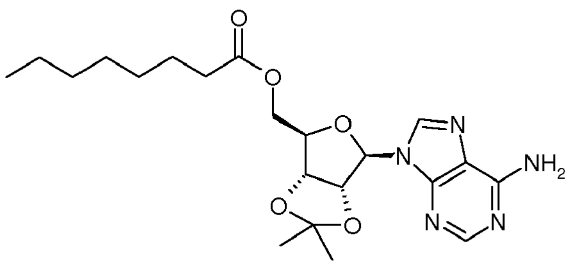

Composé C : 2',3'-isopropylidène-5'-biotinoyle-adénosine

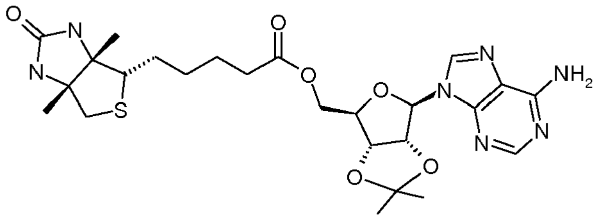

Composé D : 2',3'-isopropylidène-5'-éthyl-adénosine

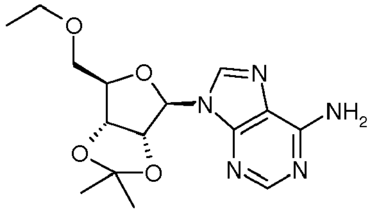

Composé E : 2',3'-isopropylidène-5'-octyle-adénosine

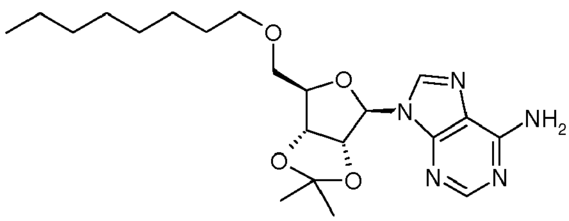

Composé F: 2',3'-diméthyl-5'-butanoyle-adénosine

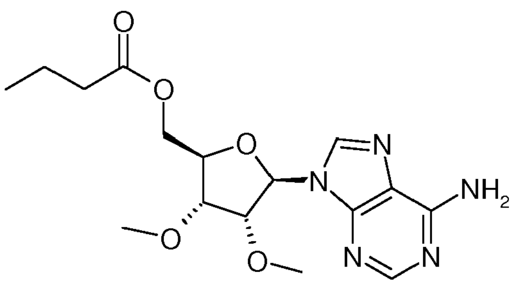

Comme composés connus faisant partie des composés de formule (I), on peut citer :

Composé G : 2',3'-isopropylidène-5'-acétyl-adénosine (n ° CAS 15888-38-7)

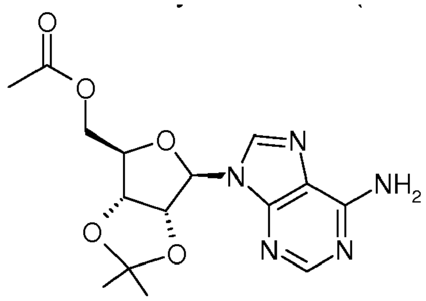

La quantité de dérivé d'adénosine utilisable selon l'invention est bien entendu fonction de l'effet recherché et peut donc varier dans une large mesure.

Pour donner un ordre de grandeur, on peut utiliser ces dérivés en une quantité représentant de 0,01 % à 10% du poids total de la composition, préférentiellement en une quantité représentant de 0,05% à 5% du poids total de la composition, plus préférentiellement en une quantité représentant de 0,1% à 2% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses. Ce milieu est avantageusement cosmétiquement acceptable, c'est-à-dire qu'il n'entraîne pas de démangeaisons, de picotements ou de rougeurs susceptibles de détourner l'utilisateur de la composition, et qu'il présente un aspect, une odeur et un toucher agréables.

Cette composition peut se présenter sous toutes les formes galéniques normalement utilisées dans le domaine cosmétique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les huiles, les cires, les émulsionnants, les gélifiants, les polymères filmogènes, les conservateurs, les parfums, les charges, les filtres UV, les bactéricides, les absorbeurs d'odeur, les matières colorantes, les actifs hydrophiles ou lipophiles, les extraits végétaux, les antioxydants. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et co-émulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants / épaississants hydrophiles, on peut citer en particulier les polymères carboxyvinyliques (carbomer), les copolymères acryliques tels que les copolymères d'acrylates/alkylacrylates, les polyacrylamides, les

polysaccharides, les gommes naturelles et les argiles, et, comme gélifiants / épaississants lipophiles, on peut citer les argiles modifiées comme les bentones, les sels métalliques d'acides gras, et la silice hydrophobe.

Comme actifs, il sera avantageux d'introduire dans la composition utilisée selon l'invention au moins un composé choisi parmi : les agents desquamants ; les agents hydratants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants et/ou les agents dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les hydroxyacides ; les hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium, en particulier les gluconates ; et leurs mélanges.

Comme indiqué précédemment, la composition selon l'invention peut également renfermer des agents photo-protecteurs actifs dans l'UVA et/ou l'UVB, sous forme de composés organiques ou inorganiques, ces derniers étant éventuellement enrobés pour les rendre hydrophobes.

Les agents photo-protecteurs organiques peuvent notamment être choisis parmi : les anthranilates, en particulier l'anthranilate de menthyle; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les agents photo-protecteurs inorganiques sont de préférence constitués d'oxyde de zinc et/ou de dioxyde de titane, de préférence de taille nanométrique, éventuellement enrobés d'alumine et/ou d'acide stéarique.

La composition selon l'invention est avantageusement destinée à être appliquée sur les zones du visage et/ou du front marquées par des rides d'expression, et/ou sur les personnes présentant des rides d'expression.

Les rides concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

L'invention sera maintenant illustrée par les exemples non limitatifs suivants. Dans ces exemples, les quantités sont indiquées en pourcentage pondéral.

**Exemple 1 :** Synthèse du 2',3'-isopropylidène-5'-biotinoyle-adénosine (Composé C)

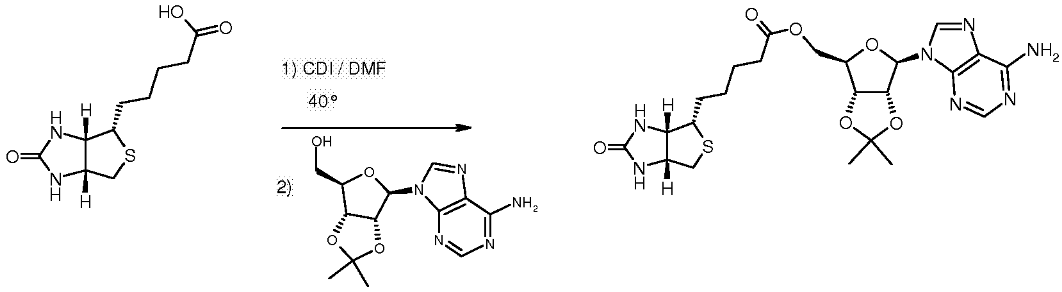


Dans un tricol, sous azote, on a introduit 150 ml de diméthylformamide, 5 g de biotine puis 4 g de carbodiimidazole (noté CDI). On a chauffé à 40 °C pendant 1 heure, puis on a additionné 6,3 g de l'isopropylidèneadénosine commercial, puis 5 mg d'amidure de sodium. La solution a été chauffée à 40 °C pendant 24 heures.
On a distillé le diméthylformamide (noté DMF) sans dépasser 40 °C, on a additionné 200 ml de dichlorométhane au résidu. La phase organique a été lavée 3 fois par 150 ml d'eau, séchée avec du sulfate de sodium et évaporée sous vide à sec. Le résidu a été purifié sur colonne de silice, éluant dichlorométhane puis dichlorométhane 95 / Méthanol 5 pour conduire à un produit solide, qui a été repris dans du dichlorométhane et précipité par du diéthyle éther.
Le précipité obtenu a été filtré, lavé à l'éther, séché sous vide.
Rendement = 50 %

Analyses :

RMN DMSO $^{1}H$ $^{13}C$ $^{2}D$: Spectres conformes
Analyse Elémentaire conforme : C 51.3 ; H 5.89 ; N 18.2 ; O 19.03 ; S 5.92

**Exemple 2 :** Synthèse du 2',3'-isopropylidène-5'-butanoyle-adénosine (Composé A) Ce composé a été préparé selon un mode opératoire similaire à celui de l'exemple 1 en utilisant l'acide butyrique à la place de la biotine.

**Exemple 3 :** Synthèse du 2',3'-isopropylidène-5'-octanoyle-adénosine (Composé B) Ce composé est préparé selon un mode opératoire similaire à celui de l'exemple 1 en utilisant l'acide octanoïque à la place de la biotine.

**Exemple 4 :** Synthèse du 2',3'-isopropylidène-5'-éthyl-adénosine (Composé D)

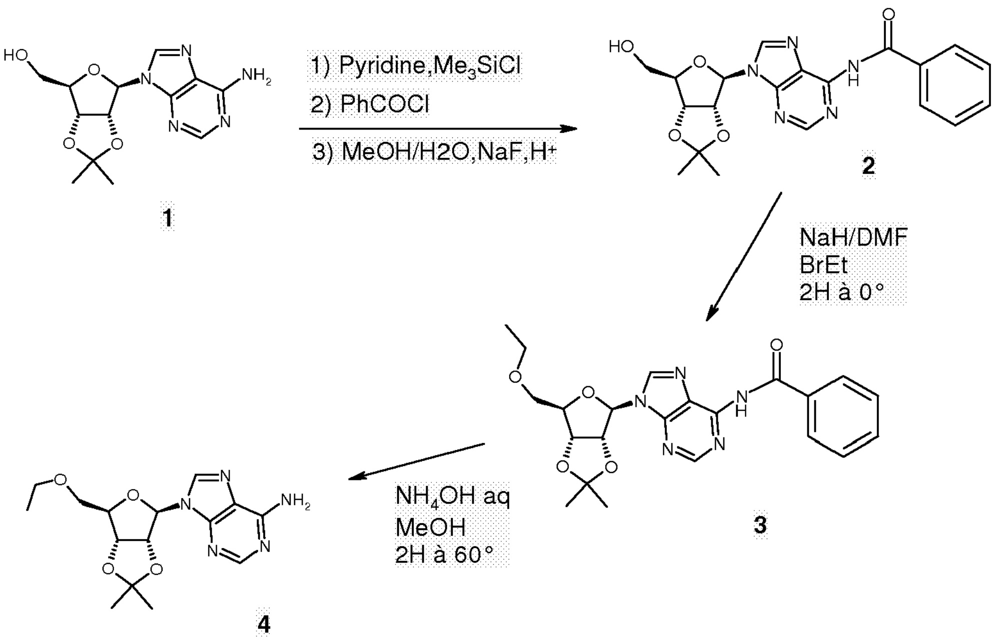


1 2 3 4

a) A une solution d'isopropylidène adénosine (1) (4g, 13 mmoles) et 4-(diméthylamino) pyridine (50mg) dans 20ml de pyridine à 0 °C, on a additionné goutte à goutte le chlorotriméthylsilane (1.76 g, 16.3mmoles).
Le mélange a été agité 10mn à 0 °C, puis 1 h à température ambiante.
On a additionné le chlorure de benzoyle (1.51 ml, 13 mmoles), agité 10 mn à 0 °C.
Après agitation 2 heures à température ambiante , 30 ml de $MeOH/H_2O$ 6 /4, NaF 1g, et chlorure de tétrabutylammonium (100 mg) ont été ajoutés, puis on a laissé réagir 1 nuit à température ambiante.
Le mélange a été dilué par 40 ml d'eau glacée et le pH a été ajusté à 2.5 par environ 40 ml d'HCl 5N ; après extraction par $CH_2Cl_2$ (4X50ml ), les phases organiques ont été lavées par 2M HCl (20ml) puis par une solution saturée de $NaHCO_3$ (30 ml), puis par de l'eau, et séchées sur $Na_2SO_4$.
Après évaporation du solvant, le résidu a été purifié sur colonne de silice, éluant $CH_2Cl_2$/Acétone 3/1.
On a obtenu 2g du composé (2) sous forme de solide blanc.

b) Une solution du composé (2) (500mg ,1.2 mmoles) dans 10 ml de DMF a été refroidie à 0 °C, on a ajouté 245 mg de NaH à 60 % dans l'huile (6 mmoles) et agité 30 mn ; 105 μl de bromoéthane a été ajouté, et on a laissé réagir 2 heures sans enlever le bain de glace ;
Le mélange a été traité par addition de 15 ml d'une solution saturée en $NH_4Cl$, puis extrait par $CH_2$ $Cl_2$. Les phases organiques ont été lavées par de l'eau puis par de l'eau saturée en NaCl, séchées sur $Na_2SO_4$ et évaporées.
On a obtenu 650 mg du composé (3) (correspondant au composé D) sous forme d'huile qui a été utilisé sans autre purification pour l'étape suivante.

c) A une solution de composé (3) (600mg, 1.4 mmoles) dans 20 ml de MeOH, on a additionné 25 ml d'une solution aqueuse à 20% de $NH_3$ (140 mmoles).
La solution a été chauffée à 60 °C pendant 2 heures., puis après refroidissement évaporée.
Le résidu a été purifié sur gel de silice ($CH_2Cl_2$ / Acétone 3/1)
On a obtenu 200 mg du composé (4) sous forme d'un solide blanc.
Rendement sur les 2 dernières étapes : 45%
RMN DMSO d6 $^1H$ $^{13}C$ : Spectres conformes

**Exemple 5**: Synthèse du 2',3'-isopropylidène-5'-octyle-adénosine (Composé E)

Ce composé a été préparé selon un mode opératoire similaire à celui de l'exemple 4 en utilisant du bromure d'octyle à la place du bromure d'éthyle.

**Exemple 6** : Mise en évidence de l'effet dermo-décontractant des composés utilisés selon l'invention.

a) Principe du test

Le principe de ce test consiste à étudier l'effet du produit à tester sur un modèle de derme équivalent constitué d'une matrice de collagène ensemencée par des fibroblastes humains normaux.
Ces conditions sont destinées à mimer *in vitro* les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

b) Protocole

Deux séries de dermes équivalents attachés contenant des fibroblastes humains normaux sont préparés : une série témoin sans aucun traitement, et une série traitée par le composé à tester (10 μM). L'expérience est répétée trois fois.
Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans composé | 45% |
| Sérum de Veau Foetal : | 10% |
| NaOH (0.1N): | 5% |
| Acide acétique (1/1000) : | 4% |
| Collagène : | 26% |
| Fibroblastes : | 10% |

Le derme équivalent traité diffère du derme équivalent témoin en ce que l'on y ajoute 10 μM du composé à tester.
Le collagène utilisé est du collagène de type I (solution commerciale). Il est extrait peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.
Le protocole est le suivant : dans un tube à centrifuger de 50 ml conservé dans la glace pilée, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de 1,5x $10^5$ cellules pour 1 ml de milieu de culture.
On ajoute alors lentement, contre la paroi du tube de façon à observer l'apparition d'un nuage blanchâtre, un mélange volume/volume de collagène dans de l'acide acétique au 1 /1000.
L'ensemble est alors mélangé avec précaution et réparti dans les puits d'une plaque de culture de 12 puits (type Costar référence 3512) à raison de 2ml de mélange par puits. La concentration cellulaire finale est de $3.10^4$ cellules/derme équivalent, avec une concentration finale en collagène de 1 mg/ml. La plaque de culture est alors placée dans un incubateur à 37°C avec 5% de $CO_2$.
Une fois formés après polymérisation du collagène, les dermes équivalents sont laissés adhérents au support de culture pendant 3 jours puis détachés du support pour que la contraction puisse démarrer. Ces dermes équivalents détachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.
L'évaluation de la contraction spontanée des dermes équivalents traité (avec le composé à tester) et témoin (sans composé à tester) est réalisée en mesurant leur surface, aux différents temps mentionnés après le début de la contraction spontanée.
Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp\text{-}Si)/Sp \times 100$$

où :

'Sp' représente la surface d'un puits de la plaque de culture ; elle correspond à la surface totale du derme équivalent avant contraction
'Si' représente la surface du derme équivalent à l'instant i de la cinétique de contraction.

**c) Résultats :**

Les composés C (exemple 1), D (exemple 4) et E (exemple 5) et G (2',3'-isopropylidène-5'-acétyl-adénosine ; composé commercialisé par la société SIGMA sous la référence I 4127) décrits précédemment ont été testés selon le protocole décrit précédemment, et comparés à l'adénosine et à l'isopropylidène adénosine.
On a obtenu les résultats suivants :

| Composé testé | % contraction |
|---|---|
| C | - 30 |
| D | -21 |
| E | - 26 |
| G | - 30 |
| Adénosine | - 15 |
| Isopropylidène adénosine | 0 |

Les composés C, D, E et G réduisent la contraction des fibroblastes d'au moins 21 % en moyenne sur la durée de l'expérience (testé à 10 μM), par rapport au témoin.
Ces composés ont donc un effet dermo-décontractant significatif qui est supérieur à celui de l'adénosine (réduction de 15 %). L'isopropylidène adénosine n'a pas d'effet dermo-décontractant..
Les composés C et G sont particulièrement préférés en raison de leur bon résultat obtenu.

**Exemple 7 : Composition de soin de la peau**

On prépare une émulsion huile-dans-eau ayant la composition suivante :

| | |
|---|---|
| Monostéarate de glycérol | 6,0% |
| Alcool stéarylique | 4,0% |
| Huile de vaseline | 10,0% |
| Huile de silicone | 5,0% |
| 5'-acétyl 2',3'-isopropylidène adénosine (composé G) | 1,0% |
| Glycérine | 8,0% |
| Polymère carboxyvinylique type Carbopol | 0,3% |
| Conservateurs | 0,4% |
| Parfum | 0,5% |
| triéthanolamine | 0,3% |
| Eau qsp | 100% |

Après application de la crème quotidiennement, les rides du visage sont estompées.
Le composé G peut être remplacé par le composé A ou B.

**Exemple 8 : Composition de soin de la peau**

On prépare un gel pour le soin du visage ayant la composition suivante :

| | |
|---|---|
| Composé C (exemple 1) | 0,1 % |
| Hydroxypropylcellulose (Klucel H vendu par la société Hercules) | 1,00 % |

(suite)

| | | |
|---|---|---|
| Antioxydant | | 0,05 % |
| Isopropanol | | 40,00 % |
| Conservateur | | 0,30 % |
| Eau | qsp | 1 00 % |

Ce gel est obtenu par mélange des constituants dans l'eau avec l'ajout, en dernier lieu du gélifiant.
Ce gel peut être appliqué deux fois par jour, il est particulièrement adapté à une application le matin car ne laisse pas la peau grasse. On constate une diminution des rides après l'application du gel quotidiennement.
Le composé C peut être remplacé par le composé F ou G ou D ou E.

## Revendications

1. Utilisation cosmétique d'au moins un dérivé d'adénosine de formule (I) :

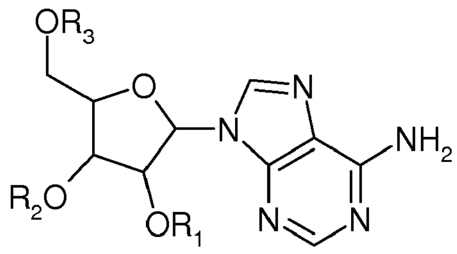

(I)

dans laquelle :

- (a) $R_1$ et $R_2$ identiques désignent un radical hydrocarboné linéaire saturé en $C_1$-$C_6$ ou insaturé en $C_2$-$C_6$ , ou ramifié en $C_3$-$C_6$ saturé ou insaturé, ou forment ensemble, avec les atomes d'oxygène auxquels ils sont rattachés un radical isopropylidène ;
et $R_3$ désigne :

(i) un groupe -$COR_4$ avec $R_4$ désignant un radical hydrocarboné linéaire saturé en $C_1$-$C_9$ ou insaturé en $C_2$-$C_9$ , ou ramifié en $C_3$-$C_9$ saturé ou insaturé, éventuellement substitué par au moins un groupement choisi parmi -OR', -NR'R", -COOR', -CONR'R", -CF3, -F , -$OCF_3$, -CN, -$NO_2$;
ou (ii) un groupe ester issu de la biotine ;

ou
- (b) $R_1$ et $R_2$ forment ensemble, avec les atomes d'oxygène auxquels ils sont rattachés, un radical isopropylidène ; et $R_3$ désigne un radical hydrocarboné linéaire saturé en $C_1$-$C_{10}$ ou insaturé en $C_2$-$C_{10}$, ou ramifié en $C_3$-$C_{10}$ saturé ou insaturé, éventuellement substitué par au moins un groupement choisi parmi -OR', -NR'R", - COOR', -CONR'R", -$CF_3$, -F , -$OCF_3$, -CN, -$NO_2$;

R' et R" désignant un atome d'hydrogène, un radical hydrocarboné linéaire saturé en $C_1$-$C_6$ ou insaturé en $C_2$-$C_6$ , ou ramifié en $C_3$-$C_6$ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en $C_1$-$C_6$ ou insaturé en $C_2$-$C_6$, ou ramifié en $C_3$-$C_6$ saturé ou insaturé ;
et leurs sels, isomères optiques et solvates,
comme agent pour lutter contre les rides, notamment les rides d'expression, et/ou décontracter la peau et/ou détendre les traits de la peau.

2. Utilisation selon la revendication précédente, **caractérisée par le fait que** le composé (I) est tel que :

- $R_1$ et $R_2$ forment ensemble, avec les atomes d'oxygène auxquels ils sont rattachés, un radical isopropylidène ;
- $R_3$ désigne :

(i) un radical hydrocarboné linéaire saturé en $C_1$-$C_{10}$ ou insaturé en $C_2$-$C_{10}$, ou ramifié en $C_3$-$C_{10}$, saturé ou insaturé ;
ou (ii) un groupe -$COR_4$ avec $R_4$ désignant un radical hydrocarboné linéaire en $C_1$-$C_9$ ou ramifié en $C_3$-$C_9$, saturé ou insaturé ;
ou (iii) un groupe ester issu de la biotine.

**3.** Utilisation selon l'une quelconque des revendications précédentes, **caractérisée par le fait que** le composé (I) est tel que :

- $R_1$ et $R_2$ forment ensemble, avec les atomes d'oxygène auxquels ils sont rattachés, un radical isopropylidène;
- $R_3$ désigne :

(i) un radical hydrocarboné linéaire en $C_1$-$C_{10}$;
ou (ii) un groupe -$COR_4$ avec $R_4$ désignant un radical hydrocarboné linéaire saturé en $C_1$-$C_9$;
ou (iii) un groupe ester issu de la biotine.

**4.** Utilisation selon l'une quelconque des revendications précédentes, **caractérisée par le fait que** le composé (I) est tel que :

$R_1$ et $R_2$ forment ensemble, avec les atomes d'oxygène auxquels ils sont rattachés, un radical isopropylidène ;
$R_3$ désigne :

- un groupe -$COR_4$ avec $R_4$ désignant un radical hydrocarboné linéaire en $C_1$-$C_9$ ;
- ou un groupe ester issu de la biotine.

**5.** Utilisation selon la revendication 1, **caractérisée par le fait que** le composé (I) est choisi parmi :

- le 2',3'-isopropylidène-5'-butanoyle-adénosine ;
- le 2',3'-isopropylidène-5'-octanoyle-adénosine ;
- le 2',3'-isopropylidène-5'-biotinoyle-adénosine ;
- le 2',3'-isopropylidène-5'-éthyl-adénosine ;
- le 2',3'-isopropylidène-5'-octyle-adénosine ;
- le 2',3'-diméthyl-5'-butanoyle-adénosine ;
- le 2',3'-isopropylidène-5'-acétyl-adénosine.

**6.** Procédé de traitement cosmétique de la peau, notamment d'une peau ridée, en particulier la peau du visage et/ou du front, comprenant l'application topique sur ladite peau d'une composition comprenant, dans un milieu physiologiquement acceptable, au moins un dérivé d'adénosine de formule (I) tel que défini selon l'une quelconque des revendications précédentes.

**7.** Composition comprenant, dans un milieu physiologiquement acceptable, au moins un dérivé d'adénosine de formule (II) :

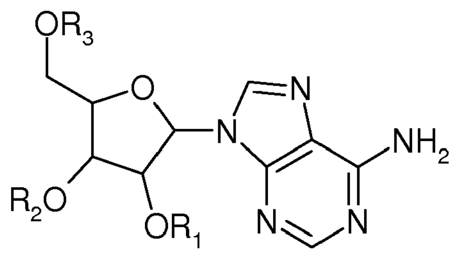

(II)

dans laquelle :

- (a) $R_1$ et $R_2$ identiques désignent un radical hydrocarboné linéaire saturé en $C_1$-$C_6$ ou insaturé en $C_2$-$C_6$, ou ramifié en $C_3$-$C_6$ saturé ou insaturé, ou forment ensemble, avec les atomes d'oxygène auxquels ils sont rattachés un radical isopropylidène;
  et $R_3$ désigne :

  (i) un groupe -$COR_4$ avec $R_4$ désignant un radical hydrocarboné linéaire saturé en $C_2$-$C_9$ ou insaturé en $C_2$-$C_9$, ou ramifié en $C_3$-$C_9$ saturé ou insaturé, éventuellement substitué par au moins un groupement choisi parmi -OR', -NR'R", -COOR', -CONR'R", -CF3, -F , -$OCF_3$, -CN, -$NO_2$;
  ou (ii) un groupe ester issu de la biotine ;

  ou
- (b) $R_1$ et $R_2$ forment ensemble, avec les atomes d'oxygène auxquels ils sont rattachés, un radical isopropylidène ; et $R_3$ désigne un radical hydrocarboné linéaire saturé en $C_1$-$C_{10}$ ou insaturé en $C_2$-$C_{10}$, ou ramifié en $C_3$-$C_{10}$ saturé ou insaturé, éventuellement substitué par au moins un groupement choisi parmi -OR', -NR'R", - COOR', -CONR'R", -$CF_3$, -F , -$OCF_3$, -CN, -$NO_2$ ;

R' et R" désignant un atome d'hydrogène, un radical hydrocarboné linéaire saturé en $C_1$-$C_6$ ou insaturé en $C_2$-$C_6$, ou ramifié en $C_3$-$C_6$ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en $C_1$-$C_6$ ou insaturé en $C_2$-$C_6$ , ou ramifié en $C_3$-$C_6$ saturé ou insaturé ;
et leurs sels, isomères optiques et solvates.

8. Composition selon la revendication précédente, **caractérisée par le fait que** le composé (II) est tel que :

   $R_1$ et $R_2$ forment ensemble, avec les atomes d'oxygène auxquels ils sont rattachés, un radical isopropylidène;
   $R_3$ désigne :

   (i) un radical hydrocarboné linéaire saturé en $C_1$-$C_{10}$ ou insaturé en $C_2$-$C_{10}$, ou ramifié en $C_3$-$C_{10}$ saturé ou insaturé ;
   ou (ii) un groupe -$COR_4$ avec $R_4$ désignant un radical hydrocarboné linéaire en $C_2$-$C_9$ ou ramifié en $C_3$-$C_9$, saturé ou insaturé;
   ou (iii) un groupe ester issu de la biotine.

9. Composition selon l'une quelconque des revendications 7 et 8, **caractérisée par le fait que** le composé (II) est tel que :

   $R_1$ et $R_2$ forment ensemble, avec les atomes d'oxygène auxquels ils sont rattachés, un radical isopropylidène;
   $R_3$ désigne :

   (i) un radical hydrocarboné linéaire en $C_1$-$C_{10}$;
   ou (ii) un groupe -$COR_4$ avec $R_4$ désignant un radical hydrocarboné linéaire saturé en $C_2$-$C_9$;
   ou (iii) un groupe ester issu de la biotine.

10. Composition selon l'une quelconque des revendications 7 à 9, **caractérisée par le fait que** le composé (II) est tel que :

    $R_1$ et $R_2$ forment ensemble, avec les atomes d'oxygène auxquels ils sont rattachés, un radical isopropylidène ;
    $R_3$ désigne :

    - un groupe -$COR_4$ avec $R_4$ désignant un radical hydrocarboné linéaire en $C_2$-$C_9$ ;
    - ou un groupe ester issu de la biotine.

11. Composition selon la revendication 7, **caractérisée par le fait que** le composé (II) est choisi parmi :

    - le 2',3'-isopropylidène-5'-butanoyle-adénosine ;
    - le 2',3'-isopropylidène-5'-octanoyle-adénosine ;

- le 2',3'-isopropylidène-5'-biotinoyle-adénosine ;
- le 2',3'-isopropylidène-5'-éthyl-adénosine ;
- le 2',3'-isopropylidène-5'-octyle-adénosine ;
- le 2',3'-diméthyl-5'-butanoyle-adénosine.

**12.** Composition selon l'une quelconque des revendications 7 à 11, **caractérisée par le fait qu'**elle constitue une composition cosmétique ou dermatologique.

**13.** Composition selon l'une quelconque des revendications 7 à 14, **caractérisée par le fait qu'**elle comprend un ingrédient choisi parmi les huiles, les cires, les émulsionnants, les gélifiants, les polymères filmogènes, les conservateurs, les parfums, les charges, les filtres UV, les bactéricides, les absorbeurs d'odeur, les matières colorantes, les actifs hydrophiles ou lipophiles, les extraits végétaux, les antioxydants.

**14.** Composés de formule (III) :

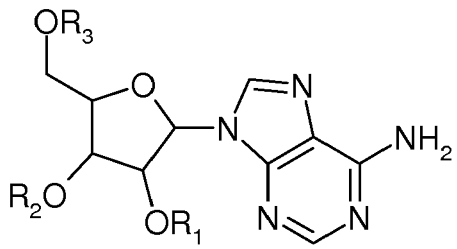

(III)

dans laquelle :

- (a) $R_1$ et $R_2$ identiques désignent un radical hydrocarboné linéaire saturé en $C_1$-$C_6$ ou insaturé en $C_2$-$C_6$ , ou ramifié en $C_3$-$C_6$ saturé ou insaturé, ou bien encore forment ensemble, avec les atomes d'oxygène auxquels ils sont rattachés un radical isopropylidène ;
- $R_3$ désigne :

(i) un groupe -$COR_4$ avec $R_4$ désignant un radical hydrocarboné linéaire en $C_3$-$C_9$ ou ramifié en $C_3$-$C_9$, saturé ou insaturé, éventuellement substitué par au moins un groupement choisi parmi -OR', -NR'R", -CONR'R", -CF3, -F , -$OCF_3$, -CN, -$NO_2$ $R_4$ ne désignant pas un groupe 2-amino éthyle ;
ou (ii) un groupe ester issu de la biotine ;

ou
- (b) $R_1$ et $R_2$ forment ensemble, avec les atomes d'oxygène auxquels ils sont rattachés, un radical isopropylidène ; et $R_3$ désigne un radical hydrocarboné linéaire en $C_2$-$C_{10}$ ou ramifié en $C_3$-$C_{10}$, saturé ou insaturé, éventuellement substitué par au moins un groupement choisi parmi -NR'R", -COOR', -CONR'R", -$CF_3$, -F , -$OCF_3$, - CN, -$NO_2$;

R' et R" désignant un atome d'hydrogène, un radical hydrocarboné linéaire saturé en $C_1$-$C_6$ ou insaturé en $C_2$-$C_6$, ou un radical ramifié en $C_3$-$C_6$ saturé ou insaturé, éventuellement substitué par au moins un groupement choisi parmi -OZ, -NZZ', - COOZ, Z et Z' désignant, indépendamment l'un de l'autre, un atome d'hydrogène ou radical hydrocarboné linéaire saturé en $C_1$-$C_6$ ou insaturé en $C_2$-$C_6$ , ou ramifié en $C_3$-$C_6$ saturé ou insaturé,
et leurs sels, isomères optiques et solvates,

**15.** Composés selon la revendication précédente, **caractérisés par le fait que**:

$R_1$ et $R_2$ forment ensemble, avec les atomes d'oxygène auxquels ils sont rattachés, un radical isopropylidène;
$R_3$ désigne :

(i) un radical hydrocarboné linéaire en $C_2$-$C_{10}$ ou ramifié en $C_3$-$C_{10}$, saturé ou insaturé ;
ou (ii) un groupe -$COR_4$ avec $R_4$ désignant un radical hydrocarboné linéaire en $C_3$-$C_9$ ou ramifié en $C_3$-$C_9$,

saturé ou insaturé ;
ou (iii) un groupe ester issu de la biotine.

**16.** Composés selon l'une quelconque des revendications 14 et 15, **caractérisés par le fait que** :

$R_1$ et $R_2$ forment ensemble, avec les atomes d'oxygène auxquels ils sont rattachés, un radical isopropylidène; $R_3$ désigne :

(i) un radical hydrocarboné linéaire en $C_2$-$C_{10}$;
ou (ii) un groupe -$COR_4$ avec $R_4$ désignant un radical hydrocarboné linéaire saturé en $C_3$-$C_9$;
ou (iii) un groupe ester issu de la biotine.

**17.** Composés selon l'une quelconque des revendications 14 à 16, **caractérisés par le fait que** :

$R_1$ et $R_2$ forment ensemble, avec les atomes d'oxygène auxquels ils sont rattachés, un radical isopropylidène; $R_3$ désigne :

- un groupe -$COR_4$ avec $R_4$ désignant un radical hydrocarboné linéaire en $C_3$-$C_9$;
- ou un groupe ester issu de la biotine.

**18.** Composés selon la revendication 14, **caractérisés par le fait qu'**ils sont choisis parmi les composés suivants :

- le 2',3'-isopropylidène-5'-butanoyle-adénosine ;
- le 2',3'-isopropylidène-5'-octanoyle-adénosine ;
- le 2',3'-isopropylidène-5'-biotinoyle-adénosine ;
- le 2',3'-isopropylidène-5'-éthyl-adénosine ;
- le 2',3'-isopropylidène-5'-octyle-adénosine ;
- le 2',3'-diméthyl-5'-butanoyle-adénosine.

Office européen des brevets

# RAPPORT DE RECHERCHE EUROPEENNE

Numéro de la demande
EP 07 10 5862

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 1 424 064 A (OREAL [FR]) 2 juin 2004 (2004-06-02) * revendications 1-6 * ----- | 1-18 | INV. C07H19/16 A61Q19/08 A61K8/49 |
| Y | EP 1 273 285 A2 (BASF AG [DE]) 8 janvier 2003 (2003-01-08) * revendications 1-8 * ----- | 1-18 | |
| Y | WO 01/43704 A1 (AVON PROD INC [US]) 21 juin 2001 (2001-06-21) * page 3 - page 5, ligne 10; revendications 1-28 * ----- | 1-18 | |
| Y | WO 00/24365 A (UNIV MASSACHUSETTS [US]) 4 mai 2000 (2000-05-04) * revendications 1-53 * ----- | 1-18 | |
| Y | EP 0 399 909 A1 (SANOFI SA [FR]) 28 novembre 1990 (1990-11-28) * revendications 1-10 * ----- | 1-18 | |

DOMAINES TECHNIQUES RECHERCHES (IPC)

C07H
A61Q
A61K

Le présent rapport a été établi pour toutes les revendications

| Lieu de la recherche | Date d'achèvement de la recherche | Examinateur |
|---|---|---|
| Munich | 6 août 2007 | Kyriakakou, Georgia |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................................
& : membre de la même famille, document correspondant

4
EPO FORM 1503 03.82 (P04C02)

**ANNEXE AU RAPPORT DE RECHERCHE EUROPEENNE RELATIF A LA DEMANDE DE BREVET EUROPEEN NO.** EP 07 10 5862

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-08-2007

| Document brevet cité au rapport de recherche | Date de publication | Membre(s) de la famille de brevet(s) | Date de publication |
|---|---|---|---|
| EP 1424064 A | 02-06-2004 | AT 363888 T | 15-06-2007 |
| | | FR 2847469 A1 | 28-05-2004 |
| EP 1273285 A2 | 08-01-2003 | CN 1395918 A | 12-02-2003 |
| | | DE 10132338 A1 | 16-01-2003 |
| | | JP 2003040729 A | 13-02-2003 |
| | | US 2003068349 A1 | 10-04-2003 |
| WO 0143704 A1 | 21-06-2001 | AR 027195 A1 | 19-03-2003 |
| | | AU 774676 B2 | 01-07-2004 |
| | | AU 2094601 A | 25-06-2001 |
| | | BR 0008214 A | 30-10-2001 |
| | | CA 2364300 A1 | 21-06-2001 |
| | | CN 1364073 A | 14-08-2002 |
| | | DE 60031836 T2 | 28-06-2007 |
| | | EP 1152731 A1 | 14-11-2001 |
| | | HK 1042044 A1 | 08-06-2007 |
| | | JP 2003516950 T | 20-05-2003 |
| | | MX PA01006553 A | 14-03-2002 |
| | | PL 349174 A1 | 01-07-2002 |
| | | US 2002006383 A1 | 17-01-2002 |
| WO 0024365 A | 04-05-2000 | AU 1231000 A | 15-05-2000 |
| | | CA 2347979 A1 | 04-05-2000 |
| | | EP 1126812 A1 | 29-08-2001 |
| | | JP 2002528400 T | 03-09-2002 |
| | | US 6423327 B1 | 23-07-2002 |
| EP 0399909 A1 | 28-11-1990 | CA 2017265 A1 | 23-11-1990 |
| | | DE 69002539 D1 | 09-09-1993 |
| | | DE 69002539 T2 | 23-12-1993 |
| | | DK 399909 T3 | 27-09-1993 |
| | | ES 2060098 T3 | 16-11-1994 |
| | | FR 2647342 A1 | 30-11-1990 |
| | | JP 3011009 A | 18-01-1991 |
| | | JP 8022813 B | 06-03-1996 |
| | | PT 94110 A | 08-01-1991 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004037159 A


### Littérature non-brevet citée dans la description


- **J.D. CARRUTERS et al.** *J. Dermatol. Surg. Oncol.,* 1992, vol. 18, 17-21
- **POPPE, L et al.** Synthesis and characterization of (5'-deoxyadenosin-5'-yl)cobalamin (='adenosylcobalamin') analogs mimicking the transition-state geometry of coenzyme-B12-dependent rearrangements. *Helvetica Chimica Acta,* 1993, vol. 76 (6), 2367-83
- **JONES, A. S. et al.** Synthetic analogs of polynucleotides. VII. Syntheses of 5'-O-acryloylnucleosides and copolymers of these with other acryloyl compounds. *Journal of the Chemical Society [Section] C: Organic,* 1971, 3183-7
- **MORNET, D. et al.** The reaction of myosin with a bromoalkyl analog of adenosine triphosphate. *FEBS Letters,* 1977, vol. 84 (2), 362-6
- **HUBER GERHARD.** esters of adenosine with organic and inorganic. *acids; Chem. Ber.,* 1956, vol. 89, 2853-62
- **TAKEMOTO, K. et al.** Nucleic acid analogs: their specific interaction and applicability. *Polymeric Materials Science and Engineering,* 1988, vol. 58, 250-3
- **PURKAYASTHA, BHUPESH C. ; BHATTACHARYYA, S. N.** Use of Ca oxalate monohydrate in the investigation of rare earth and thorium activities. *J. Indian. Chem. Soc.,* 1957, vol. 34, 427-33
- **PETERLI, STEFAN et al.** Nitrostyrene derivatives of adenosine 5'-glutarates as selective inhibitors of the epidermal growth factor receptor protein tyrosine kinase. *Helvetica Chimica Acta,* 1992, vol. 75 (3), 696-706
- *Helvetica Chimica Acta,* 1993, vol. 76, 2367
- **ASSELINEAU.** *Exp. Cell. Res.,* 1985, vol. 159, 536-539
- *Models in dermatology,* 1987, vol. 3, 1-7